(19) Europäisches Patentamt
European Patent Office
Office européen des brevets

(11) Publication number: 0 408 654 B1

# EUROPEAN PATENT SPECIFICATION

(45) Date of publication of patent specification: 24.05.95

(51) Int. Cl.⁶: C07C 231/00, C07C 233/65

(21) Application number: 89905013.2

(22) Date of filing: 29.03.89

(86) International application number:
PCT/US89/01297

(87) International publication number:
WO 89/09766 (19.10.89 89/25)

(54) PROCESS FOR THE PREPARATION OF 2,4,6-TRIIODO-5-AMINO-N-ALKYLISOPHTHALAMIC ACID.

(30) Priority: 06.04.88 US 178245

(43) Date of publication of application:
23.01.91 Bulletin 91/04

(45) Publication of the grant of the patent:
24.05.95 Bulletin 95/21

(84) Designated Contracting States:
AT BE CH DE FR GB IT LI LU NL SE

(56) References cited:
US-A- 3 145 197

Indian Drugs Pharm., vol.15,35-38B, (1980)

CHEMICAL ABSTRACTS, vol. 95, no. 15, 12
October 1981, page 631, abstract no. 132
428s, Columbus, Ohio, US; G. B. SINGH et al,
"Study on radiopaque iothalamic acid - a
comparative evaluation of its synthesis"

CHEMICAL ABSTRACTS, vol. 66, no. 7, 13
February 1967, page 2704, abstract no.
28549x, Columbus, Ohio, US; K. DIERBACH et
al, "Monomethyl amide of 2,4,6-triodo-
5-aminoisophthalic acid"; & DD-A-49 852

(73) Proprietor: MALLINCKRODT, INC.
Post Office Box 5840
St. Louis, MI 63134 (US)

(72) Inventor: CROSS, Gregory, D.
Post Office Box 5840
St. Louis, MO 63134 (US)
Inventor: CHAPMAN, Robert, C.
Post Office Box 5840
St. Louis, MO 63134 (US)

(74) Representative: Kraus, Walter, Dr. et al
Patentanwälte Kraus, Weisert & Partner
Thomas-Wimmer-Ring 15
D-80539 München (DE)

## Description

This invention relates to the synthesis of 2,4,6-triiodo-5-amino-N-alkylisophthalamic acid, and more particularly to an improved process for enhancing yields and improving the quality of the iodinated product.

2,4,6-triiodo-5-amino-N-alkylisophthalamic acid, or a salt or ester thereof, is a useful intermediate in the manufacture of X-ray contrast media. As described, for example, in Hoey patent 3,145,197, 5-acetamido-N-alkyl-2,4,6-triiodoiosphthalamic acid compounds are produced by treatment of 2,4,6-triiodo-5-amino-N-isophthalamic acid with an acylating agent such as a lower acyl halide or a lower alkanoic acid in the presence of a catalyst such as sulfuric acid or perchloric acid. In accordance with the scheme described in the Hoey patent, 5-nitroisophthalic acid is first converted to its dialkyl ester and one of the ester groups is then selectively hydrolyzed by careful treatment in a suitable solvent with one equivalent of a strong base such as sodium or potassium hydroxide. The monoester is reacted with a primary lower alkylamine to produce 5-nitro-N-alkylisophthalamic acid and the latter intermediate is subjected to catalytic hydrogenation to produce 5-amino-N-alkylisophthalamic acid commonly referred to as the "reduced half amide" or "RHA".

The RHA is triiodinated by reaction with a source of an iodine halide, preferably a source of iodine monochloride such as potassium iododichloride ($KICl_2$). In accordance with the Hoey process, the iodination reaction is effected with a modest net excess of iodinating agent, typically in hydrochloric acid solution. However, while the net overall charge of iodinating agent is in excess, the Hoey process involves first charging all or a substantial portion of the RHA to an aqueous reaction medium, and then adding the iodinating agent over a period of time. Thus, throughout most of the reaction period, there is a substantial excess of RHA in the reaction zone. In one embodiment described by Hoey, the entire RHA charge is first dissolved in a hydrochloric acid medium and the iodinating agent thereafter added thereto. In another embodiment, RHA is first reacted with less than a stoichiometrically equivalent amount of potassium iododichloride in aqueous suspension and, after several hours, sodium hydroxide and the remainder of the potassium iododichloride are added and reaction carried to completion.

The product of the reaction has generally been found to contain a fraction of mono- and di-iodinated species, thereby detracting from both product yield and product quality.

Because the RHA is typically dissolved in a hydrochloric acid medium preparatory to the addition of the iodinating agent, and because hydrochloric or other hydrogen halide acid is, in any event, a product of the reaction, the methods previously known to the art have involved conducting at least a substantial portion of the reaction at acid concentrations sufficiently high that the pH of the reaction medium is negative. Such pH conditions inhibit the progress of the reaction, thus requiring the use of an ultimate excess of the iodine halide source to drive the reaction to completion. Since the iodine halide source is not practicably recoverable from the reaction medium, the excess is effectively lost, with a resultant adverse impact on manufacturing cost. Moreover, even with an excess of iodinating reagent, the reaction is not always driven fully to completion so that the quality of the product may be less than desired.

As the reaction between RHA and iodinating agent progresses, the iodinated product compound precipitates from the reaction mixture as a crystalline solid. Acidification at the end of the reaction period precipitates the triiodo product remaining in solution. This product is recovered from the reaction mass by filtration or centrifugation. The purity of iodinated reaction product and yield obtained thereof are dependent on the efficiency of this separation. In the conventional process, some difficulty has been experienced with effective separation of the product crystals from the reaction medium mother liquor. This has detracted from the yield commercially achievable in the manufacture of X-ray contrast media from the 2,4,6-triiodo-5-amino-N-alkylisophthalamic acid produced by iodination of RHA.

Indian Drugs Pharm. Ind. 15, 35-38B (1980) describes the synthesis of isophthalamic acid as a potent X-ray contrast agent. In particular, the synthesis of 5-Amino-2,4,6-triiodo-N-methylisophthalamic acid is disclosed.

There has been a need in the art for an improved process which affords improved yields and produces a higher purity 2,4,6-triiodo-5-amino-N-alkylisophthalamic acid product.

Among the several objects of the present invention, therefore, may be noted the provision of an improved process for the manufacture of 2,4,6-triiodo-5-amino-N-alkylisophthalamic acid; the provision of such a process which affords improved yields; the provision of such a process which provides a product of enhanced quality; the provision of a process which provides favorable kinetics and improved productivity; and the provision of a process which facilitates manufacture of 2,4,6-triiodo-5-amino-N-alkylisophthalamicacid, and the X-ray contrast media for which it is an intermediate, at relatively low manufacturing cost.

Briefly, therefore, the present invention is directed to a process for the preparation of a compound selected from the group consisting of 2,4,6,-triiodo-5-amino-N-alkylisophthalamic acid, salts thereof and

2

esters thereof, comprising reaction of a substrate selected from the group consisting of 5-amino-N-alkylisophthalamic acid, salts thereof and esters thereof with an iodine halide in an aqueous reaction medium, the improvement which comprises adding said substrate and a source of said iodine halide to said reaction medium at such respective rates that, at any instant substantially throughout the addition cycle, said substrate is present in stoichiometric excess over said iodine halide, but the arithmetic difference between the cumulative amount of said substrate that has been added to said medium at said instant, expressed as a proportion of the total ultimate charge of said substrate, and the cumulative amount of said source of iodine halide that has been added to said medium at said instant, expressed as a proportion of the total ultimate charge of said source of iodine halide, does not exceed about 10%, and making an initial adjustment of the pH of said reaction medium to a value between about 0 and about 3 before commencing the iodination reaction.

The present invention is further directed to a process for the preparation of a compound selected from the group consisting of 2,4,6-triiodo-5-amino-N-alkylisophthalamic acid, salts thereof and esters thereof, comprising reaction of a substrate selected from the group consisting of 5-amino-N-allylisophthalamic acid, salts thereof, and esters thereof, with an iodine halide in an aqueous reaction medium, the improvement which comprises carrying out the reaction in the presence of an alkaline buffer composition, the proportion of said alkaline buffer composition being sufficient so that the pH of said reaction medium is maintained at between about 0 and about 3 during the course of the reaction and

adding said substrate and a source of said iodine halide to said reaction medium at such respective rates that, at any instant substantially throughout the addition cycle, said substrate is present in stoichiometric excess over said iodine halide, but the arithmetic difference between the cumulative amount of said substrate that has been added to said medium at said instant, expressed as a proportion of the total ultimate charge of said substrate, and the cumulative amount of said source of iodine halide that has been added to said medium at said instant, expressed as a proportion of the total ultimate charge of said source of iodine halide, does not exceed about 10%.

In accordance with the present invention, it has been found that limiting the unreacted RHA content of the iodination reaction system promotes conversion of that substrate to its 2,4,6-triiodo species, thereby minimizing the mono and di-iodo species in the final reaction mixture and enhancing the yield realized in the process. Moreover, it has been demonstrated that this improvement in conversion to the triiodo species is achieved without any significant increase in the formation of azo compound by products. Additionally, it has been found that, by maintaining the instantaneous excess of RHA below about 10%, high conversion to 2,4,6-triiodo-5-amino-N-alkylisophthalamic acid is achieved without a significant ultimate net excess of iodine halide. Thus, for example, by simultaneously adding substrate and iodine halide to an aqueous reaction medium at such respective rates that the instantaneous excess of RHA never exceeds about 10%, the reaction may be driven essentially to completion by the ultimate addition of a cumulative excess of iodine halide over RHA of only about 1%.

Several slightly varying computations may be used to determine the instantaneous excess of RHA. For example, the instantaneous excess of RHA may be considered as the difference, at any instant substantially throughout the addition cycle, between the cumulative number of equivalents of RHA that have been added to the reaction medium at that instant vs. the cumulative number of equivalents of iodine halide source that have been added to the reaction medium at that instant, expressed as a proportion of the total ultimate charge of iodine halide source over the addition cycle. However, since the total ultimate reactor charges of RHA and iodine halide source are generally equivalent stoichiometrically, the instantaneous excess of RHA is preferably defined by the arithmetic difference between the cumulative amount of RHA that has been delivered to the reactor at a given instant, expressed as a proportion of the total ultimate RHA charge, and the cumulative amount of iodine halide source that has been delivered at that instant, expressed as a proportion of the total ultimate iodine halide charge.

Whichever basis of computation is used, the instantaneous excess of RHA should fall in the range of between 0 and about 10%, preferably between about 2% and about 10%. This result is achieved by simultaneous addition (co-addition) of the reactants to the reaction medium and carefully monitoring the proportion of each reactant charged (or the net excess of RHA present in the medium), either on a continuous basis, or at frequent discrete intervals of time. It will be understood, of course, that where the ultimate charges of RHA and ICl are essentially equivalent, as is the case in the preferred embodiments of the process of the invention, a 2-10% RHA excess cannot be maintained entirely throughout the addition period. However, the desired excess may be maintained through substantially the entire period by, for example, stretching out the ICl addition for 5 to 10 minutes longer than the RHA addition, and maintaining the 2-10% excess until that last 5-10 minutes.

3

When the reaction is carried out by co-addition of reactants, the amount of hydrochloric acid charged to the reaction medium can be minimized, thereby reducing the usage of this raw material. Minimizing the amount of the HCl charge also contributes to control of the reaction pH at a level above 0, thus enhancing the kinetics of the iodination reaction and helping to drive it to completion even in the absence of any significant net ultimate excess of iodine halide. Thus, it has been found that, when the reaction is carried out by co-addition as described above, the amount of HCl added to the system can be limited to that sufficient to establish an initial pH of no greater than about 3 in the reaction system. During the reaction, acid is preferably not added to the reaction system. The pH is preferably adjusted to about 0.3 to 0.7 after the completion of the reaction to facilitate separation of the iodinated product by crystallization. Typically, a small amount of HCl is added for this purpose.

It has further been discovered that iodination of a substrate comprising an 5-amino-N-alkylisophthalamic acid (RHA), or its esters or salts, is promoted by the presence of an alkaline buffer composition in the reaction medium. Hydrogen halide, produced as a by-product of the reaction of an iodine halide with the substrate, is neutralized by the buffer, thereby maintaining the pH of the reaction medium at between about 0 and about 2. Control of the pH in this range essentially eliminates the inhibitory effect otherwise caused by the generation of HCl or other hydrogen halide during the reaction. When the pH is maintained in the 0 to 2 range, enhancement of the reaction kinetics is sufficient that the triiodination reaction can be carried fully to completion without the necessity of using any stoichiometric excess of the source of iodine monohalide. Because the reaction is brought to completion, the product is substantially free of partially iodinated intermediates, and thus product quality is further improved. In turn, this product may be converted to commercially valuable X-ray contrast media in accordance with the process of the aforesaid Hoey patent, and the superior quality of the iodinated RHA intermediate conduces to enhanced quality of the contrast media product as well.

Improved kinetics of reaction also allows a shortened iodination batch cycle, with consequent gain in productivity. An incremental gain in reaction rate is achieved through the reduction in HCl charge associated with the co-addition of RHA and iodinating agent. However, by itself, co-addition does not eliminate the need for at least a slight net excess of iodinating agent in the total ultimate charge of reactants to the reaction vessel. By control of pH in the 0-2 range with an alkaline buffer, the excess of iodinating agent may be essentially eliminated, and the reaction driven to completion in a reasonably short batch cycle at a temperature of 75-85°C. Increased productivity and reduced consumption of iodinating reagent provide significant economies in the manufacturing cost of the triiodo intermediate and the final X-ray contrast media product.

Preferably, the alkaline buffer composition is an alkali metal acetate such as sodium acetate. However, amonium hydroxide as well as a variety of inorganic salts of strong bases and weak acids can be used. For example, the alkaline buffer composition may comprise an alkali metal salt of phosphoric acid or an alkali metal salt of citric acid. Alkali metal salts of propionic and other alkanoic acids may also be used, but these are less preferred because of their relatively high cost. Whatever alkaline buffer composition is used, it is incorporated in the reaction medium in a proportion sufficient to maintain the pH of the reaction medium between about 0 and about 3 during the course of the iodination reaction.

Ammonium hydroxide has been found highly effective in decreasing the digest period for the reaction to go to completion. For instance, by providing two discrete pH adjustments with ammonium hydroxide during co-addition of substrate and iodine halide, the reaction may be brought to completion in 4 hours at 80°C. Incorporation of sodium acetate allows the pH to be maintained in the 1-2.5 range throughout the addition of reactants, and permits the reaction to be completed in 3 hours. 98.5% purity iodinated product is obtained from the reaction.

The iodinating reagent is iodine chloride or another iodine halide. Typically an iodine halide source is provided by adding both molecular iodine and another molecular halogen to an alkali metal halide solution. Thus, for example, molecular iodine and chlorine gas may be added to a solution of sodium chloride or potassium chloride, yielding either sodium iododichloride or potassium iododichloride, each of which is a source of iodine monochloride. Preparation of $NaICl_2$ or $KICl_2$ in this fashion is well known to those skilled in the art.

In carrying out the preferred process of the invention, an aqueous substrate solution containing 5-amino-N-alkylisophthalamic acid and an aqueous iodine halide solution are added simultaneously to an aqueous reaction medium in a reaction vessel provided with an agitator. The aqueous reaction medium may be established simply by the initial mixing of the two reactant solutions, after which the process proceeds by continued co-addition to that medium. Preferably, however, an initial charge of water, or of an acidified solution of RHA, is introduced into the reaction vessel to establish the aqueous medium before co-addition commences. If the initial charge is distilled water, addition of the iodinating agent charge solution is begun just slightly ahead of the addition of substrate charge solution so as to be certain that the RHA is not exposed to a pH above about 3. If the initial charge is an acidified RHA solution, the amount of the initial charge is controlled so that it does not contain more than about 10% of the total ultimate RHA charge. Conveniently, the substrate solution contains between about 0.02 and about 2 moles per liter of RHA and the iodine halide solution contains between about 0.05 and about 5 moles per liter of iodine halide or source thereof. At standard dilutions, the substrate charge solution may typically contain 0.1-0.3 moles/liter RHA, and the iodine halide charge solution may typically contain 0.2-0.5 equivalents/liter iodine halide source.

Where an initial water charge or RHA solution is introduced into a reaction vessel, this initial charge is preferably heated to an elevated temperature, for example in the range of 50 to 80°C before co-addition begins. Thereafter simultaneous introduction of the substrate solution and iodine halide solution to the reaction vessel is carried out and completed over a period of about 1 hour, during which the contents of the vessel are stirred to produce a homogeneous charge mixture. Agitation is continued and this mixture is maintained at an elevated temperature, typically in the range of 75 to 100°C, to complete the reaction.

The alkaline buffer composition may be introduced into the reaction medium either prior to or simultaneously with the introduction of reactant solutions. Preferably, however, the buffer composition is premixed with the substrate charge solution before it is mixed with the iodine halide solution.

Where the alkaline buffer composition is an alkali metal acetate, it is preferably prepared in situ by simultaneously adding glacial acetic acid and an aqueous solution of alkali metal hydroxide to the reaction medium or to the substrate charge solution. Preferably, the alkali metal hydroxide solution has a strength of between about 25% and about 70% by weight, most preferably about 50% by weight, alkali metal hydroxide. In situ preparation of the alkali metal acetate in this fashion facilitates plant operations since both alkali metal hydroxide solutions and glacial acetic acid are readily available liquid materials which are easily handled, thereby avoiding the necessity of mixing solid alkali metal acetate with other liquid process materials.

As the iodination reaction progresses, product 2,4,6-triiodo-5-amino-N-alkylisophthalamic acid is precipitated from the aqueous reaction mixture. The progress of the reaction may be followed by analysis of samples, preferably by high pressure liquid chromatography. At the conclusion of the reaction, an alkali metal bisulfite or other halogen scavenger is added to quench any free iodine halide remaining in the system, after which the reaction mixture is cooled and adjusted to pH of about 0.5 by addition of hydrochloric acid. Hydrochloric acid addition effects precipitation of residual product from the aqueous phase. Thereafter the reaction mixture is filtered or centrifuged for recovery of product, and the filter or centrifuge cake is washed with water and dried.

It has been found that the separation of iodinated product compound crystals from the acidified reaction medium is significantly improved if the reaction is run in a relatively dilute system. In accordance with the conventional process, the the total amount of RHA added to the reaction medium has been typically equivalent to a concentration of 0.05 to 0.15 moles per liter final reaction mass, while the amount of ICl added has been equivalent to a concentration in the neighborhood of 0.15-0.75 moles per liter, thereby resulting in the production of 2,4,6-triiodo-5-amino-N-alkylisophthalamic acid at a concentration in the range of 0.05 to 0.15 moles per liter in the slurry reaction mass. In accordance with the present invention, it has been discovered that separation is substantially facilitated, and the purity of the resultant crystalline product enhanced, if the iodinated product compound is produced in a concentration of between about 0.02 and about 0.04 moles per liter. This result may be achieved either through the use of relatively dilute reactant solutions, e.g., a substrate solution having a concentration of between about 0.02 and about 0.08, preferably about 0.02 to about 0.04, moles per liter and an iodinating agent solution having an iodine halide source concentration of between about 0.05 and about 0.1 moles per liter, or by introducing a substantial initial charge of water into the reaction vessel before the addition of reactant solutions is commenced. In either case the sum of the amounts of substrate and iodinated product preferably does not exceed about 0.08 moles/liter in the reaction mixture at any time during the cycle.

The following examples illustrate the invention.

Example 1

An RHA charge solution was prepared by adding glacial acetic acid (29 ml) and a 35°Be' sodium hydroxide solution (50 ml) to a 0.1536 g/ml solution of 5-amino-N-methylisophthalamic acid (260 ml; 0.206 mole RHA). The pH of the RHA charge solution was 6.5 and the total volume was 380 ml.

Water (1193 ml) was charged to a stirred tank reaction vessel and heated therein to a temperature of 74°C. Thereafter about 7.5% of the RHA charge solution (i.e., about 28.5 ml) was added to the reaction vessel, followed by an amount of hydrochloric acid sufficient to adjust the pH in the vessel to 1.55. After addition of HCl, the remainder of the RHA charge solution and an iodine monochloride charge solution (0.356 g/ml ICl in NaCl solution; 285 ml; 0.625 moles ICl) were added simultaneously to the reaction vessel over a period of about 2 hours. The schedule of co-addition of charge solutions and the pH of the contents of the reaction vessel during the course of the addition are set forth in Table 1. After addition of the charge solutions was completed, the resulting mixture was heated under agitation for 3 hours, after which the pH was 0.97. The reaction mixture was cooled to 65°C and sodium bisulfite (1.6 g) was added thereto. The bisulfite treated reaction mixture was cooled to 40°C and the pH was adjusted to 0.5 with 37% HCl. Precipitated product 2,4,6-triiodo-5-amino-N-methyl-isophthalamic acid was recovered by filtration. The cake was washed with water (200 ml) and dried in an oven at 95°C for three days. Yield was 114.29 g.

Table 1

| Time | RHA left (ml) | % RHA remaining to be added | ICl Remaining to be added (ml) | % ICl left to be added | % difference | pH |
|---|---|---|---|---|---|---|
| 8:45 | 351.5 | 92.5 | 285 | 100 | 7.5 | 1.55 |
| 8:50 | 335 | 88.16 | 272 | 95.44 | 7.28 | 1.45 |
| 9:00 | 304 | 80 | 246 | 86.32 | 6.32 | 1.32 |
| 9:05 | 285 | 75 | 233 | 81.75 | 6.75 | 1.30 |
| 9:10 | 270 | 71.1 | 220 | 77.19 | 6.09 | 1.34 |
| 9:21 | 236 | 62.11 | 196 | 68.77 | 6.66 | 1.40 |
| 9:30 | 206 | 54.01 | 174 | 61.05 | 6.84 | 1.38 |
| 9:40 | 178 | 46.84 | 153 | 53.68 | 6.84 | 1.43 |
| 9:50 | 145 | 38.16 | 132 | 46.32 | 8.16 | 1.48 |
| 10:00 | 115 | 30.26 | 107 | 37.54 | 7.28 | 1.47 |
| 10:10 | 83 | 21.48 | 85 | 29.82 | 7.98 | 1.48 |
| 10:20 | 52 | 13.68 | 64 | 20.46 | 8.78 | 1.50 |
| 10:30 | 20 | 5.26 | 39 | 13.68 | 8.42 | 1.50 |
| 10:40 | | | | | 7.50 | |
| 10:50 | addition complete | | | | | |

Example 2

2,4,6-triiodo-5-amino-N-methylisophthalamic acid was prepared generally in accordance with the procedure described in Example 1. In the preparation of this example, 0.1536 g/ml RHA charge solution (260 ml; 0.206 mole RHA) and 0.356 g/ml iodine monochloride charge solution (281.43 ml; 0.617 mole ICl) were utilized. The schedule of simultaneous charge solution addition is set forth in Table 2. After addition of charge solutions, the resulting mixture was heated at 90°C for three hours and then cooled to 75°C. Sodium bisulfite (1.25 g) was added to the cooled reaction mixture, after which the pH was 1.12. After bisulfite treatment of the reaction solution, 37% HCl solution was added thereto to a pH of 0.52. Dry weight of the recovered product was 114.5 g. Analysis of the product by high pressure liquid chromatography (HPLC) indicated that the product contained 97.41% 2,4,6-triido-5-amino-N-methylisophthalamic acid, 0.214% of diiodo species and 1.75% of monoiodo species.

6

Table 2

| Time | mls RHA left to be added | % RHA left to be added | ml ICI left to be added (ml) | % ICI left to be added | % difference | pH |
|---|---|---|---|---|---|---|
| | 351.5 | 92.5 | 0 | 100 | 7.5 | 1.49 |
| 9:05 | 340 | 89.47 | 270 | 96.43 | 6.96 | 1.47 |
| 9:10 | 325 | 85.53 | 261.4 | 92.89 | 7.36 | 1.41 |
| 9:20 | 304 | 80 | 246 | 87.41 | 7.41 | 1.37 |
| ppt. starting at 9:20 | | | | | | |
| 9:25 | 287 | 75.5 | 236 | 83.86 | 8.33 | 1.42 |
| 9:35 | 264 | 69.47 | 218 | 77.46 | 7.99 | 1.46 |
| 9:40 | 247 | 65 | 208 | 73.91 | 8.91 | 1.52 |
| 9:50 | 223 | 58.68 | 189 | 67.16 | 8.48 | 1.49 |
| 10:00 | 200 | 52.63 | 169 | 60.05 | 7.42 | 1.42 |
| 10:10 | 170 | 44.74 | 150 | 53.30 | 8.50 | 1.53 |
| 10:25 | 133 | 35 | 122 | 43.35 | 8.35 | 1.58 |
| 10:35 | 107 | 28.16 | 103 | 36.60 | 8.44 | 1.58 |
| 10:50 | 67 | 17.63 | 75 | 26.65 | 9.02 | 1.68 |
| | 67 | 17.63 | 70 | 24.87 | 7.24 | 1.54 |
| 11:00 | 44 | 11.58 | 55 | 19.54 | 7.96 | 1.64 |
| 11:15 | - | 0 | | | | 1.60 |
| 11:20 | | | | 0 | | 1.14 |
| 11:40 | T @ 92°C | | | | | |

## Example 3

2,4,6-triiodo-5-amino-N-methylisophthalamic acid was prepared generally in accordance with the procedure described in Example 2. In this example, however, the initial water charge to the reaction vessel was 1100 ml and the water was heated to 85°C before addition of charge solutions was commenced. RHA charge solution (7.5% of total; 28.5 ml) was then charged and 37% HCl added to a pH of 1.48. Next, a portion of the iodine monochloride solution (7.5% of total; 20 ml) was added and the resulting mixture was agitated at 85°C for 10-15 minutes, after which crystallization had begun. Simultaneous RHA and ICI charge solution addition was then carried out in accordance with the schedule set forth in Table 3. After co-addition of charge solutions was completed, the resulting mixture was heated to 92°C and maintained at that temperature for three hours. The reaction solution was then cooled to 75°C and sodium bisulfite (0.89 g) was added. After bisulfite treatment, the solution was cooled to 35-40°C and the pH adjusted to 0.49 by addition of 37% HCl (35 ml). The pH was subsequently observed to rise to about 0.6, and another portion of 37% HCl (15 ml) was added to bring the pH down to 0.5. The crystalline precipitate product was recovered by filtration, and the cake was washed with water (200 ml) and dried at 95°C over a weekend. The dry weight of the product was 113.84. Analysis of the product by HPLC indicated that it contained 97.76% by weight 2,4,6-triido-5-amino-N-methylisophthalamic acid, 1.13% by weight monoiodo species, and 0.27% by weight diiodo species.

Table 3

| Time | RHA left(ml) | % RHA left | ICl left to be added (ml) | % ICl left to be added | % difference | pH |
|---|---|---|---|---|---|---|
| | 351.5 | 92.5 | 281.43 | 100 | 7.5 | 1.48 |
| 7.5 __ 37% HCl | | | | | | |
| 9:13 | 351.5 | 92.5 | 261.43 | 92.89 | .39 | .97 |
| 9:20 | started ppt. | | | | | |
| 9:25 | started RHA/ICl to maintain 3.5%-4% RHA excess. | | | | | |
| 9:36 | 322 | 84.74 | 250 | 88.83 | 4.09 | 1.11 |
| 9:43 | 310 | 81.58 | 239 | 84.92 | 3.34 | 1.09 |
| 9:49 | 290 | 76.32 | 226 | 80.30 | 3.98 | 1.13 |
| 9:57 | 265 | 69.74 | 208 | 73.91 | 4.17 | 1.16 |
| 10:11 | 230 | 60.53 | 181 | 64.31 | 3.78 | 1.19 |
| 10:19 | 203 | 53.42 | 162 | 51.56 | 4.14 | 1.20 |
| 10:27 | 185 | 48.68 | 146 | 51.87 | 3.19 | 1.20 |
| 10:38 | 154 | 40.53 | 120 | 42.64 | 2.11 | 1.19 |
| 10:44 | 130 | 34.21 | 104 | 36.95 | 2.74 | 1.16 |
| 11:00 | 110 | 28.95 | 80 | 28.43 | | 1.12 |
| | 94.73 | 24.93 | 80 | 28.47 | 3.52 | 1.20 |
| 11:12 | 83 | 21.84 | 69 | 24.52 | 2.68 | 1.23 |
| 11:20 | 53 | 13.95 | 53 | 18.83 | 4.88 | 1.36 |
| 11:35 | 30 | 7.89 | 40 | 14.21 | 6.30 | 1.45 |
| 11:45 | 0 | | 15 | | 5.32 | 1.45 |
| | | | 0 | | | 1.12 |

Comparative Example 1

Distilled water (1348 ml) was charged to a 2 liter 4-neck round bottom flask equipped with a thermometer, pH probe, subsurface RHA inlet tube, above surface iodine chloride inlet tube, stirrer and heating mantle. A thermo-watch temperature controller was provided for use in controlling the temperature of the contents of the flask. Each of the reactant solution inlet tubes was fed from a charge solution source through a Masterflex metering pump used to control the rate of addition.

The water charge was heated to a temperature of 80-82°C. Over a two hour time period thereafter, a 0.394 g/ml iodine chloride charge solution (297.09 ml; 117.05 gms; 0.7210 moles) and a 0.213 gms/ml RHA charge solution (211.17 ml.; 45 gm; 0.2318 mole) were added to the reaction flask. Introduction of the iodine chloride solution into the reaction medium was begun just prior to the addition of RHA charge solution to make certain that the pH was sufficiently low to prevent undesirable reactions. However, immediately after introduction of iodine chloride solution was begun, addition of RHA charge solution was commenced, and addition of the two charge solutions was continued at such respective rates that a modest excess of RHA over iodine chloride prevailed through the ensuing two hour period of addition. Specifically, the respective rates of addition were controlled so that, at any instant during the addition cycle, the cumulative amount of RHA that had been added to the medium, taken as a proportion of the total ultimate charge of the substrate, exceeded the cumulative amount of iodine chloride source that had been added to the medium, taken as a proportion of the total ultimate charge of the iodine chloride source, but the arithmetic difference between such proportions was maintained in the range of 0-7%.

When approximately 10% of the RHA had been charged to the reaction flask, precipitation of iodinated product compound commenced. At the conclusion of addition of the RHA and iodinated chloride charge solution, the pH of the reaction medium was in the range of 0.7-0.8. After addition of the charge solutions was complete, the reaction mass was heated to 95°C and maintained at that temperature for three hours. During this digest period, heat was removed and the stirrer stopped periodically to allow the taking of reaction mother liquid samples which were tested for completeness of reaction. A small amount of sodium bisulfite was added to each reaction sample prior to its analysis by high pressure liquid chromatography (HPLC). At the end of the three hour reaction period, the reaction mass was cooled to 70°C and treated with sodium bisulfite until the reaction mother liquor gave a negative response to starch paper. The reaction mass was then cooled to 40°C and the filter cake washed with distilled water (225 ml). The solids recovered by filtration were dried in a vacuum oven overnight at 95-100°C. Light cream crystals having a purity of 97.6-97.8% purity were obtained in a yield of 128.66 gm. Thus the percentage yield exceeded that of the conventional process by 4.28%. HPLC analysis indicated that complete reaction had been obtained and, specifically, that the levels of di- and mono-iodo species were negligible.

HPLC was run on the product without dilution and on the isolated product at a 2mg/ml level. HPLC conditions were as follows: 5 micron radial compression column, solvent A to B, 5% per minute, gradient program B, run time of 25 min., flow set 4.5 and flow 3.0.

Comparative Example 2

Using a procedure similar to that described in Comparative Example 1, a series of iodination reactions was run at varying combinations of temperature, reaction time, net ultimate excess of iodine chloride, and post reaction treatment dosage of sodium bisulfite. The results of the runs of this series are set forth in Table 4.

TABLE 4

| Exp. No. | Description | % Excess ICl | pH Digest | TIME DIGEST HRS | TEMP DIGEST °C | gm Na BISULPHITE | % Reduction in Yield vs. Exp. #39 |
|---|---|---|---|---|---|---|---|
| 39 | Co-addition, No HCl | 3.68 | .7-.8 | 3 | 95 | 2.9 | — |
| 43 | Co-addition, No HCl | 1.70 | .7-.8 | 4-1/2-5 | 80 | 1.5 | .35% |
| 45 | Co-addition, No HCl | .95 | .7-.8 | 6 | 80 | 1.17 | .48% |
| 47 | Co-addition, no HCl | .95 | .7-.8 | 4.5 | 90 | .85 | .47% |
| 53 | Co-addition, No HCl, 2 $NH_4OH$ Adjustments | .95 | 2.0 | 4 | 80 | .63 | — |
| 55 | Co-addition, No HCl, 2 $NH_4OH$ Adjustments | .95 | 2.0 | 3 | 80 | — | — |
| 56 | Co-addition, No HCl, Na acetate | .95 | 2.58 | 4 | 80 | .70 | — |
| 57 | Co-addition, No HCl, Na acetate | .95 | 2.40 | 4 | 80 | .43 | — |

Co-addition, buffer, digest time and temperature

These results demonstrate the high yields achieved with minimal excess iodine chloride when operating in accordance with the co-addition scheme of the process of the invention. Since operation under co-addition conditions at 3.68% excess ICl provides a 0.9-1.2% increase in the weight yield of the iodinated product as compared to operation at the same excess under standard operating conditions, it may be seen that co-addition permits the ICl excess to be reduced, for example, to 1% while still attaining a 0.6-0.9% absolute increase in product weight yield as compared to the standard process at the higher ICl excess.

From results such as those summarized above, the yield on ICl appear to be optimized at an approximately 1% net ultimate excess of ICl, a digest temperature of 80-92°C, and a digest period of 5 to 8 hours.

Comparative Example 3

2,4,6-triiodo-5-amino-N-methylisophthalamic acid was prepared generally in accordance with the procedure described in Example 1. The initial charge to the reaction vessel comprised water (1320 ml) and 37% hydrochloric acid (2.5 ml). The concentration of the RHA charge solution was similar to that of Example 1, but the total volume of RHA charge solution was 111.6 ml. The ICl charge solution had a strength of 0.352 g/ml and a total volume of 166.2 ml. The schedule of co-addition of charge solutions and the pH of the contents of the reaction vessel during the course of the addition are set forth in Table 5. After co-addition was completed, the mixture in the reaction vessel was heated at 95°C for two and one-half hours, after which the pH was 0.92. By additon of 37% hydrochloric acid (20 ml), the pH was adjusted to 0.62. The reaction mixture was cooled to 70°C and sodium bisulfite (0.4 g) was added. The product obtained by crystallization consisted of very light cream-colored crystals which were readily recovered by filtration. Yield was 65.29 g.

The ammonium salt of 2,4,6-triiodo-5-amino-N-methylisophthalamic acid ($NH_4$.TIA) was prepared by: dissolving a portion of the iodinated product (25 g) in water (200 ml), by adding 35° Be' sodium hydroxide solution to pH of 4.5-6.0; heating the resulting solution to 60-70°C; adding ammonium chloride (25 g) to the solution; cooling the solution to 45°C to crystallize out the $NH_4$.TIA; separating the crystals from the mother liquor by filtration; and washing the filter cake with an aliquot of ammonium chloride solution (0.2 g/ml).

Table 5

| An Example of 2X Dilution with Co-addition | | | | | | |
|---|---|---|---|---|---|---|
| Time | mls RHA Left | % RHA Left | mls ICl Left | % ICl Left | % differ. | pH |
| 8:50 | 103 | 92.5 | 166.2 | 100 | 7.5 | 1.50 |
| 9:00 | 97 | 86.9 | 155 | 93.3 | 6.3 | 1.45 |
| 9:10 | 90 | 80.7 | 143 | 86.0 | 5.4 | 1.96 |
| 9:14 | ppt started | | | | | |
| 9:20 | 84 | 75.3 | 130 | 78.2 | 2.95 | 1.46 |
| 9:30 | 74 | 66.3 | 122 | 73.4 | 7.1 | 1.46 |
| 9:40 | 69 | 61.8 | 112 | 67.38 | 5.6 | 1.34 |
| 9:50 | 62 | 55.6 | 100 | 60.17 | 4.6 | 1.40 |
| 10:00 | 56 | 50.2 | 92 | 55.36 | 5.2 | 1.39 |
| 10:10 | 50 | 44.8 | 82 | 49.34 | 4.5 | 1.39 |
| 10:20 | 44 | 39.4 | 70 | 42.18 | 2.75 | 1.30 |
| 10:26 | 38.7 | 34.7 | 70 | 42.12 | 7.45 | 1.30 |
| 10:35 | 33 | 29.6 | 63 | 37.9 | 8.35 | 1.32 |
| 10:45 | 26.5 | 23.8 | 54 | 32.5 | 8.74 | 1.41 |
| 10:54 | 18 | 16.12 | 38 | 22.86 | 6.74 | 1.30 |
| 11:40 | stopped $NH_4$OH (added 38 ml of 1:1 of 29.8% $NH_4$OH over total period) | | | | | |
| 11:40 | heat to 95°C | | | | | |
| 12:00 | T @ 95°C - 2:30 | | | | | |

In view of the above, it will be seen that the several objects of the invention are achieved and other advantageous results attained.

As various changes could be made in the above methods without departing from the scope of the invention, it is intended that all matter contained in the above description shall be interpreted as illustrative and not in a limiting sense.

**Claims**

1. In a process for the preparation of a compound selected from the group consisting of 2,4,6,-triiodo-5-amino-N-alkylisophthalamic acid, salts thereof and esters thereof, comprising reaction of a substrate selected from the group consisting of 5-amino-N-alkylisophthalamic acid, salts thereof and esters thereof with an iodine halide in an aqueous reaction medium, the improvement which comprises adding said substrate and a source of said iodine halide to said reaction medium at such respective rates that, at any instant substantially throughout the addition cycle, said substrate is present in stoichiometric excess over said iodine halide, but the arithmetic difference between the cumulative amount of said substrate that has been added to said medium at said instant, expressed as a proportion of the total ultimate charge of said substrate, and the cumulative amount of said source of iodine halide that has been added to said medium at said instant, expressed as a proportion of the total ultimate charge of said source of iodine halide, does not exceed about 10%, and making an initial adjustment of the pH of said reaction medium to a value between about 0 and about 3 before commencing the iodination reaction.

2. An improved process as set forth in claim 1 wherein said arithmetic difference is maintained at between about 2% and about 10%.

3. An improved process as set forth in claim 1 wherein the pH of the reaction medium at the beginning of the reaction is between about 2.5 and about 3.0.

4. An improved process as set forth in claim 1 wherein the pH of the reaction medium is maintained at not greater than about 3 during the course of the reaction.

5. An improved process as set forth in claim 4 wherein addition of said source of iodine halide is commenced just prior to the addition of said substrate to said medium so that said substrate is not exposed to a pH of greater than about 3 in said reaction medium.

6. An improved process as set forth in claim 1 wherein the concentration of said iodinated product compound does not exceed about 0.08 moles/liter in the reaction mass at the conclusion of the iodination reaction, said reaction mass comprising the combination of a liquid phase comprising said reaction medium and any solids precipitated from said medium during the course of the reaction.

7. An improved process as set forth in claim 6 where the concentration of said iodinated product compound does not exceed about 0.08 moles/liter in the reaction mass at any time during the iodination reaction cycle.

8. An improved process as set forth in claim 7 wherein the sum of the amounts of said substrate and said iodinated product compound added to said medium does not exceed about 0.08 moles/liter in the reaction mass at any time during said reaction cycle.

9. An improved process as set forth in claims 6 wherein the reaction is carried out in the presence of an alkaline buffer composition, the proportion of said alkaline buffer composition being sufficient so that the pH of said reaction medium is maintained at between about 0 and about 3 during the course of the reaction.

10. An improved process as set forth in claim 9 wherein said buffer composition is selected from the group consisting of alkali metal acetates, ammonium hydroxide, alkali metal phosphates and alkali metal citrates.

11. An improved process as set forth in claim 10 wherein said buffer composition comprises an alkali metal acetate.

12. An improved process as set forth in claim 11 wherein said alkali metal acetate is produced in situ by adding an alkali metal hydroxide and glacial acetic acid to said reaction medium.

13. An improved process as set forth in claim 12 wherein said alkali metal hydroxide is added in the form of an aqueous solution thereof that contains between about 25% and about 70% by weight of said alkali metal hydroxide.

14. An improved process as set forth in claim 11 wherein a substrate charge solution comprising an aqueous solution of said substrate and an iodine halide charge solution comprising an aqueous solution containing a source of said iodine halide are simultaneously added to and mixed in a reaction vessel, said alkali metal acetate being provided by introducing an alkali metal hydroxide and glacial acetic acid into said substrate charge solution before mixing thereof with said iodine halide charge solution.

15. An improved process as set forth in claim 14 wherein said alkali metal hydroxide is introduced in the form of an aqueous solution thereof that contains between about 25% and about 70% by weight of said alkali metal hydroxide.

16. An improved process as set forth in claim 10 wherein said buffer comprises ammonium hydroxide.

17. An improved process as set forth in claim 16 wherein a substrate charge solution comprising an aqueous solution of said substrate and an iodine halide charge solution comprising an aqueous solution containing a source of said iodine halide are simultaneously added to and mixed in a reaction vessel, ammonium hydroxide being incorporated into said substrate charge solution before mixing thereof with said iodine halide charge solution.

18. An improved process as set forth in claim 9 wherein the total charge of said substrate and the total charge of said iodine halide added to said reaction medium over the course of the reaction are in substantial stoichiometric equivalence.

19. An improved process as set forth in claim 9 wherein the pH of the reaction medium at the beginning of the reaction period is between about 2.5 and about 3.0.

20. An improved process as set forth in claim 9 wherein the concentration of said iodinated product compound does not exceed about 0.08 moles/liter in the reaction mass at the conclusion of the iodination reaction, said reaction mass comprising the combination of a liquid phase comprising said reaction medium and any solids precipitated from said medium during the course of the reaction.

21. In a process for the preparation of a compound selected from the group consisting of 2,4,6-triiodo-5-amino-N-alkylisophthalamic acid, salts thereof and esters thereof, comprising reaction of a substrate selected from the group consisting of 5-amino-N-alkylisophthalamic acid, salts thereof, and esters thereof, with an iodine halide in an aqueous reaction medium, the improvement which comprises carrying out the reaction in the presence of an alkaline buffer composition, the proportion of said alkaline buffer composition being sufficient so that the pH of said reaction medium is maintained at between about 0 and about 3 during the course of the reaction and
adding said substrate and a source of said iodine halide to said reaction medium at such respective rates that, at any instant substantially throughout the addition cycle, said substrate is present in stoichiometric excess over said iodine halide, but the arithmetic difference between the cumulative amount of said substrate that has been added to said medium at said instant, expressed as a proportion of the total ultimate charge of said substrate, and the cumulative amount of said source of iodine halide that has been added to said medium at said instant, expressed as a proportion of the total ultimate charge of said source of iodine halide, does not exceed about 10%

22. An improved process as set forth in claim 21 wherein said buffer composition is selected from the group consisting of alkali metal acetates, ammonium hydroxide, alkali metal phosphates and alkali metal citrates.

23. An improved process as set forth in claim 22 wherein said buffer composition comprises an alkali metal acetate.

24. An improved process as set forth in claim 23 wherein said alkali metal acetate is produced in situ by adding an alkali metal hydroxide and glacial acetic acid to said reaction medium.

**25.** An improved process as set forth in claim 24 wherein said alkali metal hydroxide is added in the form of an aqueous solution thereof that contains between about 25% and about 70% by weight of said alkali metal hydroxide.

**26.** An improved process as set forth in claim 23 wherein a substrate charge solution comprising an aqueous solution of said substrate and an iodine halide charge solution comprising an aqueous solution containing a source of said iodine halide are simultaneously added to and mixed in a reaction vessel, said alkali metal acetate being provided by introducing an alkali metal hydroxide and glacial acetic acid into said substrate charge solution before mixing thereof with said iodine halide charge solution.

**27.** An improved process as set forth in claim 26 wherein said alkali metal hydroxide is introduced in the form of an aqeous solution thereof that contains between about 25% and about 70% by weight of said alkali metal hydroxide.

**28.** An improved process as set forth in claim 21 wherein said substrate and a source of said iodine halide are added to said reaction medium in substantial stoichiometric equivalence and the reaction is carried out with substantially stoichiometrically equivalent amounts of substrate and iodine halide in the reaction medium.

**29.** An improved process as set forth in claim 28 wherein said buffer composition is selected from the group consisting of alkali metal acetates, ammonium hydroxide, alkali metal phosphates and alkali metal citrates.

**30.** An improved process as set forth in claim 29 wherein said buffer composition comprises an alkali metal acetate.

**31.** An improved process as set forth in claim 30 wherein said alkali metal acetate is produced in situ by adding an alkali metal hydroxide and glacial acetic acid to said reaction medium.

**32.** An improved process as set forth in claim 30 wherein a substrate charge solution comprising an aqueous solution of said substrate and an iodine halide charge solution comprising an aqueous solution containing said source of iodine halide are simultaneously added to and mixed in a reaction vessel, said alkali metal acetate being provided by introducing an alkali metal hydroxide and glacial acetic acid into said substrate charge solution before mixing thereof with said iodine halide charge solution.

**33.** An improved process as set forth in claim 32 wherein said alkali metal hydroxide is introduced in the form of an aqueous solution thereof that contains between about 25% and about 70% by weight of said alkali metal hydroxide.

**34.** An improved process as set forth in claim 21 wherein a substrate charge solution comprising an aqueous solution of said substrate and an iodine halide charge solution comprising an aqueous solution containing a source of said iodine halide are simultaneously added to and mixed in a reaction vessel, and thereafter the resulting mixture is maintained at a temperature of between about 90° and about 100°C to complete the triiodination of the substrate.

**35.** An improved process as set forth in claim 34 wherein an initial water charge is introduced into a reaction vessel provided with an agitator, after said water charge has been introduced said substrate charge solution and said iodine halide charge solution are simultaneously added to the reactor and the contents of the reactor are stirred with the agitator to provide a substantially homogeneous mixture, and thereafter the resulting mixture is heated to complete the triiodination of the substrate.

**36.** An improved process as set forth in claim 34 wherein said substrate charge solution contains between about 0.1 and about 0.3 moles per liter of said substrate.

**37.** An improved process as set forth in claim 36 wherein said iodine halide charge solution contains between about 0.2 and about 0.5 equivalents per liter of said source of iodine halide.

**Patentansprüche**

1. Verfahren zur Herstellung einer Verbindung, ausgewählt aus der Gruppe, bestehend aus 2,4,6-Triiod-5-amino-N-alkylisophthalamidsäure, den Salzen davon und den Estern davon, durch Umsetzung eines Substrats, ausgewählt aus der Gruppe, bestehend aus 5-Amino-N-alkylisophthalamidsäure, den Salzen davon und den Estern davon mit einem Iodhalogenid in wäßrigem Reaktionsmedium, wobei das Substrat und eine Quelle für das Iodhalogenid zu dem Reaktionsmedium in solchen entsprechenden Geschwindigkeiten zugegeben werden, daß zu irgendeinem momentanen Zeitpunkt im wesentlichen während des gesamten Additionszyklus das Substrat in stöchiometrischem Überschuß gegenüber dem Iodhalogenid vorhanden ist, aber die arithmetrische Differenz zwischen der kumulativen Menge des Substrats, die zu dem Medium bis zu dem genannten Augenblick zugegeben wurde, ausgedrückt als Verhältnis der gesamten Endbeschickung an Substrat und der kumulativen Menge der genannten Quelle an Iodhalogenid, die zu dem Medium bis zu dem momentanen Augenblick zugegeben wurde, ausgedrückt als Verhältnis der gesamten Endbeschickung an der Quelle an Iodhalogenid, nicht etwa 10% überschreitet, und wobei eine Anfangseinstellung des pH's des Reaktionsmediums auf einen Wert zwischen etwa 0 und etwa 3 erfolgt, bevor die Iodierungsreaktion begonnen wird.

2. Verbessertes Verfahren nach Anspruch 1, dadurch **gekennzeichnet**, daß die arithmetrische Differenz zwischen etwa 2 % und etwa 10 % gehalten wird.

3. Verbessertes Verfahren nach Anspruch 1, dadurch **gekennzeichnet**, daß der pH des Reaktionsmediums zu Beginn der Reaktion zwischen etwa 2,5 und etwa 3,0 liegt.

4. Verbessertes Verfahren nach Anspruch 1, dadurch **gekennzeichnet**, daß der pH des Reaktionsmediums bei nicht über etwa 3 während des Verlaufs der Reaktion gehalten wird.

5. Verbessertes Verfahren nach Anspruch 4, dadurch **gekennzeichnet**, daß die Zugabe der Quelle an Iodhalogenid gerade vor der Zugabe des Substrats zu dem Medium begonnen wird, so daß das Substrat nicht einem pH-Wert, der größer als etwa 3 ist, in dem Reaktionsmedium ausgesetzt ist.

6. Verbessertes Verfahren nach Anspruch 1, dadurch **gekennzeichnet**, daß die Konzentration der iodierten Produktverbindung nicht etwa 0,08 mol/l in der Reaktionsmasse bei Beendigung der Iodierungsreaktion überschreitet, wobei die Reaktionsmasse ein Gemisch aus flüssiger Phase, welche das Reaktionsmedium enthält, und irgendwelchen Feststoffen, die aus dem Medium im Verlauf der Reaktion ausgefallen sind, enthält.

7. Verbessertes Verfahren nach Anspruch 6, dadurch **gekennzeichnet**, daß die Konzentration der iodierten Produktverbindung nicht etwa 0,08 mol/l in der Reaktionsmasse zu irgendeinem Zeitpunkt während des Iodierungsreaktionszyklus überschreitet.

8. Verbessertes Verfahren nach Anspruch 7, dadurch **gekennzeichnet**, daß die Summe der Mengen des Substrats und der iodierten Produktverbindung, die zu dem Medium zugegeben wird, nicht etwa 0,08 mol/l in der Reaktionmasse zu irgendeiner Zeit während des Reaktionszyklus überschreitet.

9. Verbessertes Verfahren nach Anspruch 6, dadurch **gekennzeichnet**, daß die Reaktion in Anwesenheit einer alkalischen Pufferzusammensetzung durchgeführt wird, wobei das Verhältnis der alkalischen Pufferzusammensetzung ausreicht, so daß der pH des Reaktionsmediums zwischen etwa 0 und etwa 3 im Verlauf der Reaktion gehalten wird.

10. Verbessertes Verfahren nach Anspruch 9, dadurch **gekennzeichnet**, daß die Pufferzusammensetzung ausgewählt wird aus der Gruppe, bestehend aus Alkalimetallacetaten, Ammoniumhydroxid, Alkalimetallphosphaten und Alkalimetallcitraten.

11. Verbessertes Verfahren nach Anspruch 10, dadurch **gekennzeichnet**, daß die Pufferzusammensetzung ein Alkalimetallacetat umfaßt.

12. Verbessertes Verfahren nach Anspruch 11, dadurch **gekennzeichnet**, daß das Alkalimetallacetat in situ durch Zugabe eines Alkalimetallhydroxids und Eisessig zu dem Reaktionsmedium gebildet wird.

**13.** Verbessertes Verfahren nach Anspruch 12, dadurch **gekennzeichnet**, daß das Alkalimetallhydroxid in Form einer wäßrigen Lösung davon, die zwischen etwa 25 und etwa 70 Gew.-% Alkalimetallhydroxid enthält, zugegeben wird.

**14.** Verbessertes Verfahren nach Anspruch 11, dadurch **gekennzeichnet**, daß eine Substratbeschickungslösung, die eine wäßrige Lösung des Substrats umfaßt, und eine Iodhalogenidbeschickungslösung, die eine wäßrige Lösung, die eine Quelle für das Iodhalogenid enthält, umfaßt, gleichzeitig zugegeben werden und in dem Reaktor vermischt werden, wobei das Alkalimetallacetat zugeführt wird, indem ein Alkalimetallhydroxid und Eisessig in die Substratbeschickungslösung eingeleitet werden, bevor diese mit der Iodhalogenidbeschickungslösung vermischt wird.

**15.** Verbessertes Verfahren nach Anspruch 14, dadurch **gekennzeichnet**, daß das Alkalimetallhydroxid in Form einer wäßrigen Lösung davon eingeleitet wird, die zwischen etwa 25 und etwa 70 Gew.-% des Alkalimetallhydroxids enthält.

**16.** Verbessertes Verfahren nach Anspruch 10, dadurch **gekennzeichnet**, daß der Puffer Ammoniumhydroxid umfaßt.

**17.** Verbessertes Verfahren nach Anspruch 16, dadurch **gekennzeichnet**, daß eine Substratbeschickungslösung, die eine wäßrige Lösung des Substrats umfaßt, und eine Iodhalogenidbeschickungslösung, die eine wäßrige Lösung, die eine Quelle für das Iodhalogenid enthält, umfaßt, gleichzeitig zugegeben werden und in dem Reaktor vermischt werden, Ammoniumhydroxid in die Substratbeschickungslösung eingearbeitet wird, bevor diese mit der Iodhalogenidbeschickungslösung vermischt wird.

**18.** Verbessertes Verfahren nach Anspruch 9, dadurch **gekennzeichnet**, daß die Gesamtbeschickung des Substrats und die Gesamtbeschickung des Iodhalogenids, die zu dem Reaktionsmedium im Verlauf der Reaktion zugegeben werden, im wesentlichen stöchiometrisch equivalent sind.

**19.** Verbessertes Verfahren nach Anspruch 9, dadurch **gekennzeichnet**, daß der pH des Reaktionsmediums zu Beginn der Reaktionszeit zwischen etwa 2,5 und etwa 3,0 liegt.

**20.** Verbessertes Verfahren nach Anspruch 9, dadurch **gekennzeichnet**, daß die Konzentration der iodierten Produktverbindung nicht etwa 0,08 mol/l in der Reaktionsmasse bei Beendigung der Iodierungsreaktion überschreitet, wobei die Reaktionsmasse das Gemisch aus flüssiger Phase, welche das Reaktionsmedium enthält, und irgendwelchen Feststoffen, die aus dem Medium im Verlauf der Reaktion präzipitiert sind, umfaßt.

**21.** Verfahren zur Herstellung einer Verbindung, ausgewählt aus der Gruppe, bestehend aus 2,4,6-Triiod-5-amino-N-alkylisophthalamidsäure, den Salzen davon und den Estern davon, durch Umsetzung eines Substrats, ausgewählt aus der Gruppe, bestehend aus 5-Amino-N-alkylisophthalamidsäure, den Salzen davon und den Estern davon, mit einem Iodhalogenid in wäßrigem Reaktionsmedium, wobei die Reaktion in Anwesenheit einer alkalischen Pufferzusammensetzung durchgeführt wird, der Anteil der alkalischen Pufferzusammensetzung ausreicht, daß der pH des Reaktionsmediums zwischen etwa 0 und etwa 3 im Verlauf der Reaktion gehalten wird, und das Substrat und eine Quelle für das Iodhalogenid zu dem Reaktionsmedium in solchen entsprechenden Geschwindigkeiten zugegeben werden, daß zu irgendeinem Moment im wesentlichen während des Additionszyklus das Substrat in stöchimetrischem Überschuß im Vergleich zu dem Iodhalogenid vorhanden ist, aber die arithmetrische Differenz zwischen der kumulativen Menge des Substrats, die zu dem Medium bis zu dem genannten Augenblick zugegeben wurde, ausgedrückt als Verhältnis der gesamten Endbeschickung an Substrat, und der kumulativen Menge an Quelle für Iodhalogenid, die zu dem Medium bis zu dem Augenblick zugegeben wurde, ausgedrückt als Verhältnis der gesamten Endbeschickung der Quelle an Iodhalogenid, nicht etwa 10% überschreitet.

**22.** Verbessertes Verfahren nach Anspruch 21, dadurch **gekennzeichnet**, daß die Pufferzusammensetzung ausgewählt wird aus der Gruppe, bestehend aus Alkalimetallacetaten, Ammoniumhydroxid, Alkalimetallphosphaten und Alkalimetallcitraten.

EP 0 408 654 B1

23. Verbessertes Verfahren nach Anspruch 22, dadurch **gekennzeichnet**, daß die Pufferzusammensetzung ein Alkalimetallacetat umfaßt.

24. Verbessertes Verfahren nach Anspruch 23, dadurch **gekennzeichnet**, daß das Alkalimetallacetat in situ durch Zugabe eine Alkalimetallhydroxids und Eisessig zu dem Reaktionsmedium gebildet wird.

25. Verbessertes Verfahren nach Anspruch 24, dadurch **gekennzeichnet**, daß das Alkalimetallhydroxid in Form einer wäßrigen Lösung davon, die zwischen etwa 25 und etwa 70 Gew.-% an Alkalimetallhydroxid enthält, zugegeben wird.

26. Verbessertes Verfahren nach Anspruch 23, dadurch **gekennzeichnet**, daß eine Substratbeschickungslösung, welche eine wäßrige Lösung aus dem Substrat umfaßt, und eine Iodhalogenidbeschickungslösung, die eine wäßrige Lösung, welche eine Quelle für das Iodhalogenid enthält, umfaßt, gleichzeitig zugegeben werden und in dem Reaktor vermischt werden, wobei das Alkalimetallacetat durch Zugabe eines Alkalimetallhydroxids und Eisessig in die Substratbeschikkungslösung gebildet wird, bevor diese mit der Iodhalogenidbeschickungslösung vermischt wird.

27. Verbessertes Verfahren nach Anspruch 26, dadurch **gekennzeichnet**, daß das Alkalimetallhydroxid in Form einer wäßrigen Lösung davon, die zwischen etwa 25 und etwa 70 Gew.-% Alkalimetallhydroxid enthält, eingeleitet wird.

28. Verbessertes Verfahren nach Anspruch 21, dadurch **gekennzeichnet**, daß das Substrat und eine Quelle für das Iodhalogenid zu dem Reaktionsmedium in im wesentlichen stöchimetrisch equivalenten Mengen zugegeben werden und daß die Reaktion mit im wesentlichen stöchimetrisch equivalenten Mengen an Substrat und Iodhalogenid in dem Reaktionsmedium durchgeführt wird.

29. Verbessertes Verfahren nach Anspruch 28, dadurch **gekennzeichnet**, daß die Pufferzusammensetzung ausgewählt wird aus der Gruppe, bestehend aus Alkalimetallacetaten, Ammoniumhydroxid, Alkalimetallphosphaten und Alkalimetallcitraten.

30. Verbessertes Verfahren nach Anspruch 29, dadurch **gekennzeichnet**, daß die Pufferzusammensetzung ein Alkalimetallacetat umfaßt.

31. Verbessertes Verfahren nach Anspruch 30, dadurch **gekennzeichnet**, daß das Alkalimetallacetat in situ durch Zugabe eines Alkalimetallhydroxids und Eisessig zu dem Reaktionsmedium gebildet wird.

32. Verbessertes Verfahren nach Anspruch 30, dadurch **gekennzeichnet**, daß die Substratbeschickungslösung, die eine wäßrige Lösung aus dem Substrat umfaßt, und eine Iodhalogenidbeschickungslösung, die eine wäßrige Lösung, welche die Quelle für Iodhalogenid enthält, umfaßt, gleichzeitig zugegeben werden und in dem Reaktor vermischt werden, wobei das Alkalimetallacetat durch Einleiten eines Alkalimetallhydroxids und Eisessig in die Substratbeschickungslösung, bevor diese mit der Iodhalogenidbeschickungslösung vermischt wird, erzeugt wird.

33. Verbessertes Verfahren nach Anspruch 32, dadurch **gekennzeichnet**, daß das Alkalimetallhydroxid in Form einer wäßrigen Lösung davon, die zwischen etwa 25 und 70 Gew.-% Alkalimetallhydroxid enthält, eingeleitet wird.

34. Verbessertes Verfahren nach Anspruch 21, dadurch **gekennzeichnet**, daß eine Substratbeschickungslösung, die eine wäßrige Lösung aus dem Substrat umfaßt, und eine Iodhalogenidbeschickungslösung, die eine wäßrige Lösung, die eine Quelle aus Iodhalogenid enthält, umfaßt, gleichzeitig zugegeben werden und in dem Reaktor vermischt werden, und daß anschließend das entstehende Gemisch bei einer Temperatur zwischen etwa 90° und etwa 100°C zur Beendigung der Triiodierung des Substrats gehalten wird.

35. Verbessertes Verfahren nach Anspruch 34, dadurch **gekennzeichnet**, daß eine Anfangswasserbeschickung in den Reaktor, der mit einer Rührvorrichtung ausgerüstet ist, eingeleitet wird, nachdem die Wasserbeschickung eingeleitet wurde, die Substratbeschickungslösung und die Iodhalogenidbeschickungslösung gleichzeitig in den Reaktor gegeben werden, und der Inhalt des Reaktors mit der

17

Rührvorrichtung gerührt wird, wobei ein im wesentlichen homogenes Gemisch erhalten wird, und anschließend das entstehende Gemisch zur Beendigung der Triiodierung des Substrats erhitzt wird.

**36.** Verbessertes Verfahren nach Anspruch 34, dadurch **gekennzeichnet**, daß die Substratbeschickungslösung zwischen etwa 0,1 und etwa 0,3 mol/l an dem Substrat enthält.

**37.** Verbessertes Verfahren nach Anspruch 36, dadurch **gekennzeichnet**, daß die Iodhalogenidbeschikkungslösung zwischen etwa 0,2 und etwa 0,5 Equivalenten pro Liter an Quelle an Iodhalogenid enthält.

**Revendications**

**1.** Dans un procédé pour la préparation d'un composé choisi parmi l'acide 2,4,6-triiodo-5-amino-N-alkylisophtalamique, ses sels et ses esters, comprenant la réaction d'un substrat choisi parmi l'acide 5-amino-N-alkylisophtalamique, ses sels et ses esters avec un halogénure d'iode dans un milieu de réaction aqueux, le perfectionnement qui consiste à ajouter ledit substrat et une source dudit halogénure d'iode audit milieu de réaction à des débits respectifs tels qu'à tout instant, pratiquement dans tout le cycle d'addition, ledit substrat soit présent en excès stoechiométrique par rapport audit halogénure d'iode, mais que la différence arithmétique entre la quantité cumulée dudit substrat qui a été ajoutée audit milieu audit instant, exprimée en proportion de la charge finale totale dudit substrat, et la quantité cumulée de ladite source d'halogénure d'iode qui a été ajoutée audit milieu audit instant, exprimée en proportion de la charge finale totale de ladite source d'halogénure d'iode, ne dépasse pas environ 10 %, et à effectuer un réglage initial du pH dudit milieu de réaction à une valeur comprise entre environ 0 et environ 3 avant de commencer la réaction d'iodation.

**2.** Procédé perfectionné selon la revendication 1, dans lequel ladite différence arithmétique est maintenue entre environ 2 et environ 10 %.

**3.** Procédé perfectionné selon la revendication 1, dans lequel le pH du milieu de réaction au début de la réaction est compris entre environ 2,5 et environ 3,0.

**4.** Procédé perfectionné selon la revendication 1, dans lequel le pH du milieu de réaction est maintenu à pas plus de 3 pendit le cours de la réaction.

**5.** Procédé perfectionné selon la revendication 4, dans lequel l'addition de ladite source d'halogénure d'iode est commencée juste avant l'addition dudit substrat audit milieu, de sorte que ledit substrat n'est pas exposé à un pH de plus d'environ 3 dans ledit milieu de réaction.

**6.** Procédé perfectionné selon la revendication 1, dans lequel la concentration dudit produit iodé ne dépasse pas environ 0,08 mol/l dans la masse de réaction à la fin de la réaction d'iodation, ladite masse de réaction comprenant la combinaison d'une phase liquide comprenant ledit milieu de réaction et des solides éventuels précipités dans ledit milieu au cours de la réaction.

**7.** Procédé perfectionné selon la revendication 6, dans lequel la concentration dudit produit iodé ne dépasse pas environ 0,08 mol/l dans la masse de réaction à tout instant pendant le cycle de la réaction d'iodation.

**8.** Procédé perfectionné selon la revendication 7, dans lequel la somme des quantités dudit substrat et dudit produit iodé ajoutés audit milieu ne dépasse pas environ 0,08 mol/l dans la masse de réaction à tout instant pendant ledit cycle de réaction.

**9.** Procédé perfectionné selon la revendication 6, dans lequel la réaction est effectuée en présence d'une composition tampon alcaline, la proportion de ladite composition tampon alcaline étant suffisante pour que le pH dudit milieu de réaction soit maintenu entre environ 0 et environ 3 pendant le cours de la réaction.

**10.** Procédé perfectionné selon la revendication 9, dans lequel ladite composition tampon est choisie parmi les acétates de métaux alcalins, l'hydroxyde d'ammonium, les phosphates de métaux alcalins et les citrates de métaux alcalins.

**11.** Procédé perfectionné selon la revendication 10, dans lequel ladite composition tampon comprend un acétate de métal alcalin.

**12.** Procédé perfectionné selon la revendication 11, dans lequel ledit acétate de métal alcalin est produit in situ par addition d'un hydroxyde de métal alcalin et d'acide acétique glacial audit milieu de réaction.

**13.** Procédé perfectionné selon la revendication 12, dans lequel ledit hydroxyde de métal alcalin est ajouté sous la forme d'une solution aqueuse qui contient entre environ 25 et environ 70 % en poids dudit hydroxyde de métal alcalin.

**14.** Procédé perfectionné selon la revendication 11, dans lequel une solution de charge du substrat comprenant une solution aqueuse dudit substrat et une solution de charge de l'halogénure d'iode comprenant une solution aqueuse contenant une source dudit halogénure d'iode sont ajoutées simultanément et mélangées dans un récipient de réaction, ledit acétate de métal alcalin étant fourni en introduisant un hydroxyde de métal alcalin et de l'acide acétique glacial dans ladite solution de charge du substrat avant son mélange avec ladite solution de charge de l'halogénure d'iode.

**15.** Procédé perfectionné selon la revendication 14, dans lequel ledit hydroxyde de métal alcalin est introduit sous la forme d'une solution aqueuse qui contient entre environ 25 et environ 70 % en poids dudit hydroxyde de métal alcalin.

**16.** Procédé perfectionné selon la revendication 10, dans lequel ledit tampon comprend de l'hydroxyde d'ammonium.

**17.** Procédé perfectionné selon la revendication 16, dans lequel une solution de charge du substrat comprenant une solution aqueuse dudit substrat et une solution de charge de l'halogénure d'iode comprenant une solution aqueuse contenant une source dudit halogénure d'iode sont ajoutées simultanément et mélangées dans un récipient de réaction, de l'hydroxyde d'ammonium étant incorporé dans ladite solution de charge du substrat avant son mélange avec ladite solution de charge de l'halogénure d'iode.

**18.** Procédé perfectionné selon la revendication 9, dans lequel la charge totale dudit substrat et la charge totale dudit halogénure d'iode ajoutées audit milieu de réaction au cours de la réaction sont en proportions pratiquement stoechiométriques.

**19.** Procédé perfectionné selon la revendication 9, dans lequel le pH du milieu de réaction au début de la période de réaction est compris entre environ 2,5 et environ 3,0.

**20.** Procédé perfectionné selon la revendication 9, dans lequel la concentration dudit produit iodé ne dépasse pas environ 0,08 mol/l dans la masse de réaction à la fin de la réaction d'iodation, ladite masse de réaction comprenant la combinaison d'une phase liquide comprenant ledit milieu de réaction et des solides éventuels précipités dans ledit milieu au cours de la réaction.

**21.** Dans un procédé pour la préparation d'un composé choisi parmi l'acide 2,4,6-triiodo-5-amino-N-alkylisophtalamique, ses sels et ses esters, comprenant la réaction d'un substrat choisi parmi l'acide 5-amino-N-alkylisophtalamique, ses sels et ses esters avec un halogénure d'iode dans un milieu de réaction aqueux, le perfectionnement qui consiste
à mettre en oeuvre la réaction en présence d'une composition tampon alcaline, la proportion de ladite composition tampon alcaline étant suffisante pour que le pH dudit milieu de réaction soit maintenu entre environ 0 et environ 3 pendant le cours de la réaction et
à ajouter ledit substrat et une source dudit halogénure d'iode audit milieu de réaction à des débits respectifs tels qu'à tout instant, pratiquement dans tout le cycle d'addition, ledit substrat soit présent en excès stoechiométrique par rapport audit halogénure d'iode, mais que la différence arithmétique entre la quantité cumulée dudit substrat qui a été ajoutée audit milieu audit instant, exprimée en proportion de la charge finale totale dudit substrat, et la quantité cumulée de ladite source d'halogénure d'iode qui a été ajoutée audit milieu audit instant, exprimée en proportion de la charge finale totale de ladite source d'halogénure d'iode, ne dépasse pas environ 10 %.

EP 0 408 654 B1

**22.** Procédé perfectionné selon la revendication 21, dans lequel ladite composition tampon est choisie parmi les acétates de métaux alcalins, l'hydroxyde d'ammonium, les phosphates de métaux alcalins et les citrates de métaux alcalins.

**23.** Procédé perfectionné selon la revendication 22, dans lequel ladite composition tampon comprend un acétate de métal alcalin.

**24.** Procédé perfectionné selon la revendication 23, dans lequel ledit acétate de métal alcalin est produit in situ par addition d'un hydroxyde de métal alcalin et d'acide acétique glacial audit milieu de réaction.

**25.** Procédé perfectionné selon la revendication 24, dans lequel ledit hydroxyde de métal alcalin est ajouté sous la forme d'une solution aqueuse qui contient entre environ 25 et environ 70 % en poids dudit hydroxyde de métal alcalin.

**26.** Procédé perfectionné selon la revendication 23, dans lequel une solution de charge du substrat comprenant une solution aqueuse dudit substrat et une solution de charge de l'halogénure d'iode comprenant une solution aqueuse contenant une source dudit halogénure d'iode sont ajoutées simultanément et mélangées dans un récipient de réaction, ledit acétate de métal alcalin étant fourni en introduisant un hydroxyde de métal alcalin et de l'acide acétique glacial dans ladite solution de charge du substrat avant son mélange avec ladite solution de charge de l'halogénure d'iode.

**27.** Procédé perfectionné selon la revendication 26, dans lequel ledit hydroxyde de métal alcalin est introduit sous la forme d'une solution aqueuse qui contient entre environ 25 et environ 70 % en poids dudit hydroxyde de métal alcalin.

**28.** Procédé perfectionné selon la revendication 21, dans lequel ledit substrat et une source dudit halogénure d'iode sont ajoutés audit milieu de réaction en proportions pratiquement stoechiométriques et la réaction est mise en oeuvre avec des quantités pratiquement stoechiométriques du substrat et de l'halogénure d'iode dans le milieu de réaction.

**29.** Procédé perfectionné selon la revendication 28, dans lequel ladite composition tampon est choisie parmi les acétates de métaux alcalins, l'hydroxyde d'ammonium, les phosphates de métaux alcalins et les citrates de métaux alcalins.

**30.** Procédé perfectionné selon la revendication 29, dans lequel ladite composition tampon comprend un acétate de métal alcalin.

**31.** Procédé perfectionné selon la revendication 30, dans lequel ledit acétate de métal alcalin est produit in situ par addition d'un hydroxyde de métal alcalin et d'acide acétique glacial audit milieu de réaction.

**32.** Procédé perfectionné selon la revendication 30, dans lequel une solution de charge du substrat comprenant une solution aqueuse dudit substrat et une solution de charge d'halogénure d'iode comprenant une solution aqueuse contenant ladite source de l'halogénure d'iode sont ajoutées simultanément et mélangées dans un récipient de réaction, ledit acétate de métal alcalin étant fourni en introduisant un hydroxyde de métal alcalin et de l'acide acétique glacial dans ladite solution de charge du substrat avant son mélange avec ladite solution de charge d'halogénure d'iode.

**33.** Procédé perfectionné selon la revendication 32, dans lequel ledit hydroxyde de métal alcalin est introduit sous la forme d'une solution aqueuse qui contient entre environ 25 et environ 70 % en poids dudit hydroxyde de métal alcalin.

**34.** Procédé perfectionné selon la revendication 21, dans lequel une solution de charge du substrat comprenant une solution aqueuse dudit substrat et une solution de charge de l'halogénure d'iode comprenant une solution aqueuse contenant une source dudit halogénure d'iode sont ajoutées simultanément et mélangées dans un récipient de réaction, et ensuite le mélange résultant est maintenu à une température comprise entre environ 90 et environ 100 °C pour terminer la triiodation du substrat.

20

**35.** Procédé perfectionné selon la revendication 34, dans lequel une charge d'eau initiale est introduite dans un récipient de réaction muni d'un agitateur; après l'introduction de ladite charge d'eau, ladite solution de charge du substrat et ladite solution de charge de l'halogénure d'iode sont ajoutées simultanément dans le réacteur et le contenu du réacteur est agité avec l'agitateur pour donner un mélange pratiquement homogène ; et ensuite, le mélange résultant est chauffé pour terminer la triiodation du substrat.

**36.** Procédé perfectionné selon la revendication 34, dans lequel ladite solution de charge de substrat contient entre environ 0,1 et environ 0,3 mol/l dudit substrat.

**37.** Procédé perfectionné selon la revendication 36, dans lequel ladite solution de charge de l'halogénure d'iode contient entre environ 0,2 et environ 0,5 équivalent/l de ladite source d'halogénure d'iode.